(19) **Europäisches Patentamt**
**European Patent Office**
**Office européen des brevets**

(11) **EP 3 664 710 B1**

(12) # EUROPEAN PATENT SPECIFICATION

(45) Date of publication and mention
of the grant of the patent:
**19.11.2025 Bulletin 2025/47**

(21) Application number: **18759411.4**

(22) Date of filing: **03.08.2018**

(51) International Patent Classification (IPC):
**A61B 5/085** (2006.01)   **A61B 5/00** (2006.01)
**A61M 16/00** (2006.01)   **G16H 50/30** (2018.01)

(52) Cooperative Patent Classification (CPC):
**A61B 5/085; A61B 5/7275; A61M 16/0006;**
**G16H 50/30;** A61M 16/026; A61M 2016/0027;
A61M 2016/0036; A61M 2230/46

(86) International application number:
**PCT/IB2018/055857**

(87) International publication number:
**WO 2019/030632 (14.02.2019 Gazette 2019/07)**

(54) **A SYSTEM FOR THE EARLY IDENTIFICATION OF RECURRENCES OF CHRONIC OBSTRUCTIVE PULMONARY DISEASE**

SYSTEM ZUR FRÜHERKENNUNG VON REZIDIVEN VON CHRONISCH OBSTRUKTIVER LUNGENERKRANKUNG

SYSTÈME D'IDENTIFICATION PRÉCOCE DE RÉCIDIVES DE BRONCHOPNEUMOPATHIE CHRONIQUE OBSTRUCTIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.08.2017 IT 201700093172**

(43) Date of publication of application:
**17.06.2020 Bulletin 2020/25**

(73) Proprietor: **Restech S.r.l.**
**20124 Milano (IT)**

(72) Inventors:
• **GOBBI, Alessandro**
**24127 Bergamo (IT)**
• **POMPILIO, Pasquale Pio**
**20126 Milano (IT)**
• **DELLACA', Raffaele**
**22070 Valmorea (CO) (IT)**

(74) Representative: **Murgitroyd & Company**
**165-169 Scotland Street**
**Glasgow G5 8PL (GB)**

(56) References cited:
EP-A1- 2 245 985   EP-A1- 2 613 696
WO-A1-03/103493   WO-A1-2005/104944

• ANONYMOUS SWATI A ET AL: "A Study of Artifacts and Their Removal During Forced Oscillation of the Respiratory System | Annals of Biomedical Engineering", 8 January 2013 (2013-01-08), XP093274808, Retrieved from the Internet <URL:https://link.springer.com/article/10.1007/s10439-012-0735-9>
• PHAM THUY T ET AL: "Respiratory Artefact Removal in Forced Oscillation Measurements: A Machine Learning Approach", IEEE TRANSACTIONS ON BIOMEDICAL ENGINEERING, IEEE, USA, vol. 64, no. 8, 1 August 2017 (2017-08-01), pages 1679 - 1687, XP011656305, ISSN: 0018-9294, [retrieved on 20170714], DOI: 10.1109/TBME.2016.2554599
• FRAN?OIS MARCHAL ET AL: "Respiratory impedance response to a deep inhalation in children with history of cough or asthma", PEDIATRIC PULMONOLOGY., vol. 33, no. 6, 1 June 2002 (2002-06-01), US, pages 411 - 418, XP055518577, ISSN: 8755-6863, DOI: 10.1002/ppul.10093

**(Cont. next page)**

- GOBBI A ET AL: "A new telemedicine system for the home monitoring of lung function in patients with obstructive respiratory diseases", EHEALTH, TELEMEDICINE, AND SOCIAL MEDICINE, 2009. ETELEMED '09. INTERNATIONAL CONFERENCE ON, IEEE, PISCATAWAY, NJ, USA, 1 February 2009 (2009-02-01), pages 117 - 122, XP002663800, ISBN: 978-1-4244-3360-5, [retrieved on 20090213], DOI: 10.1109/ETELEMED.2009.18
- R.L. DELLACÀ ET AL.: "Detection of expiratory flow limitation in COPD using the forced oscillation technique", EUROPEAN RESPIRATORY JOURNAL., vol. 23, no. 2, 1 February 2004 (2004-02-01), DK, pages 232 - 240, XP055477726, ISSN: 0903-1936, DOI: 10.1183/09031936.04.00046804
- MICHAEL D GOLDMAN ET AL: "Within- and between-day variability of respiratory impedance, using impulse oscillometry in adolescent asthmatics", PEDIATRIC PULMONOLOGY, JOHN WILEY, NEW YORK, NY, US, vol. 34, no. 4, 1 October 2002 (2002-10-01), pages 312 - 319, XP002628720, ISSN: 8755-6863, [retrieved on 20020830], DOI: 10.1002/PPUL.10168

**Description**

The present invention refers to a system and computer program for the early identification of exacerbations in patients suffering from chronic obstructive pulmonary disease.

Chronic obstructive pulmonary disease (COPD) is a chronic respiratory disease characterized by persistent symptoms such as dyspnea, chronic coughing and expectoration and by persistent airflow limitation (GOLD 2017). Common risk factors include prolonged exposure to noxious particles and/or gases, such as cigarette smoke. The progression of COPD is characterized by stable periods interrupted by exacerbations, namely acute deteriorations of the symptoms and the underlying inflammatory process which, in the most serious cases, can require hospitalization of the patient (Vogelmeier et al., 2017).

The frequency of the exacerbation episodes has important consequences for the clinical history of the patient, accelerating functional decline of the lungs, increasing the risk of death, reducing the quality of life and increasing the social and economic costs associated with the pathology.

The evidence that early therapeutic intervention on the exacerbation episodes can help to reduce their impact on the patients' health (Wilkinson, Donaldson, Hurst, Seemungal, & Wedzicha, 2004), together with the necessity to optimize the management of patients suffering from COPD, has stimulated the development of care models based on home monitoring programs. The majority of the programs proposed are based on the use of daily questionnaires for recording worsening of the symptoms perceived by the patients in combination with medical teleconsulting systems and patient education. Although these programs have demonstrated effectiveness in reducing hospitalizations and the number of patients accessing A&E due to exacerbations of COPD (McLean et al., 2012), they have not been applied on a large scale due to the high implementation costs required.

An alternative approach consists in the combination of measurements of physiological parameters that can be performed by the COPD patient at home, without direct medical supervision, with automatic algorithms that are able to identify the exacerbations early starting from analysis of the measurements performed. The medical personnel are therefore alerted only if the algorithm has identified a suspected deterioration in the state of health of one of the COPD patients being treated who, consequently, can be immediately contacted to verify his/her state of health and/or to optimize the course of treatment.

Since said approach does not require continuous review of the measurements taken by the medical personnel, it would allow the management of a large number of patients by a restricted medical team, thus guaranteeing implementation on a large scale.

The experimental studies in which said approach has been studied used measurements of cardiac frequency and blood oxygen saturation (measured by means of portable pulsometers), alone or in combination with mechanical respiratory measurements (with portable spirometers). Said studies have not demonstrated adequate effectiveness in improving the management of patients suffering from COPD during a exacerbation (Ringbaek et al., 2015; Vianello et al., 2016).

EP2245985 A1 describes a detection monitoring device for monitoring patient to evaluate respiratory insufficiency or chronic obstructive pulmonary disease condition, with a processor controlling evaluation of a severity change indicator based on measure from signal.

EP2613696 A1 describes another system for the automatic detection of respiratory diseases and for the prediction of future acute airway instabilities.

The object of the present invention is to provide a system for early identification of exacerbations of COPD, using respiratory function parameters measured by means of the forced oscillation technique (FOT).

In accordance with the present invention, said object and others are achieved by a system according to claim 1.

Further characteristics of the invention are described in the dependent claims.

The forced oscillation technique (FOT) is a non-invasive method for measuring the mechanical properties of the airways and lungs based on the recording of pressure and flow to the patient's mouth during the application of a low-pressure external stimulus oscillating at a frequency higher than that of spontaneous breathing. (Dubois, Brody, Lewis and Burgess, 1956). This characteristic allows the measurement to be performed during spontaneous breathing, therefore making it ideal for remote monitoring applications, without supervision, of the respiratory parameters as demonstrated for example in the pilot studies of Dellacà et al. (Raffaele L. Dellacà, Gobbi, Pastena, Pedotti and Celli, 2010) and Gulotta et al. (Gulotta et al., AJRCCM, 2012).

During the FOT measurement, small oscillations in pressure (approximately 1-3 cmH2O peak-peak) at a single or composite frequency (usually between 4 and 40Hz) are sent to the patient's lungs through the opening of the airways (nose and/or mouth) by using a mouthpiece or alternative interfaces such as nasal or facial masks. The response of the respiratory system is evaluated in terms of impedance (Zrs), which is the overall ratio between the pressure at the mouth and the airflow at the oscillation frequencies. The impedance Zrs is usually divided into its real component, the resistance (Rrs), and the imaginary component, the reactance (Xrs).

Rrs and Xrs can be analysed both in the time domain, i.e. during the respiration cycle (intra-breath analysis) and in the frequency domain (frequency analysis).

In the first case (intra-breath analysis) Rrs and Xrs are calculated at each breath, as described for example in Dellacà et al. (Dellacà et al., ERJ, 2004). Rrs and Xrs can therefore be presented both for each breath or as a mean of all the breaths of a given measurement. The intra-breath analysis allows Rrs and Xrs to be used to automatically exclude some breaths from the measurement mean if they are affected by artefacts, such as swallowing, coughing, etc. An example of said algorithm is described in Gobbi et al. (Gobbi et al., IEEE Telemed, 2009). Furthermore, with respect to the frequency analysis, in the intra-breath analysis the number of frequencies contained in the pressure stimulus is usually lower; this allows improvement of the signal-noise ratio and further separation of the contribution of inspiration and expiration of both the Rrs (obtaining the inspiratory resistance, Rinsp, and expiratory resistance, Rexp, respectively) and the Xrs (obtaining the inspiratory reactance, Xinsp, and expiratory reactance, Xexp, respectively) at each stimulus frequency. The results of the inspiratory and expiratory parameters can be reported for both each breath and as a mean of the breaths without artefacts contained in the measurement itself. For example, the mean difference between Xinsp and Xexp at 5Hz within an FOT test is indicated by the symbol $\Delta$Xrs and has been shown to be associated with expiratory flow reduction (R. L. Dellacà et al., 2004), a condition that occurs in patients affected by severe or very serious COPD. Since an FOT measurement is performed during quiet breathing, from said measurement it is also possible to derive various respiratory pattern parameters, for example the tidal volume ($V_T$), the mean inspiratory and expiratory flows and times, the respiratory frequency and minute ventilation.

The characteristics and advantages of the present invention will be evident from the following detailed disclosure of a practical embodiment thereof, illustrated by way of non-limiting example in the accompanying drawings, in which:

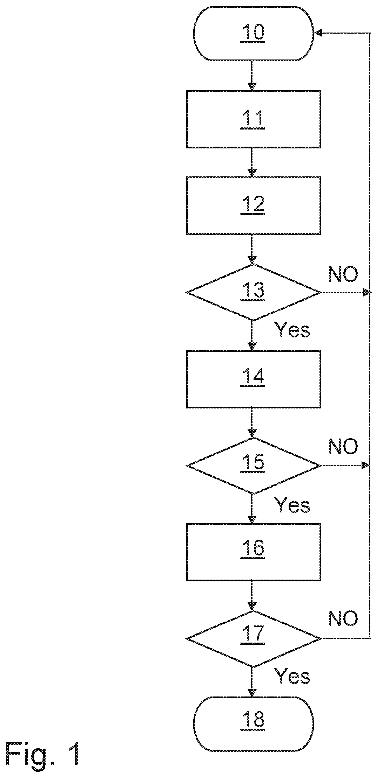

figure 1 shows a flow diagram of a method for early identification of exacerbations of COPD, in accordance with the present invention;

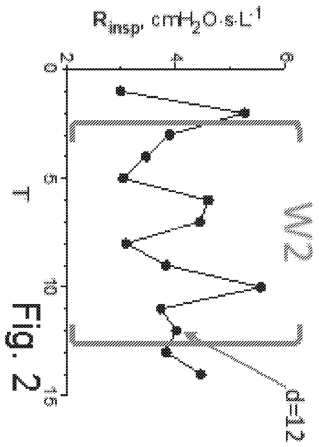

figure 2 shows a graph exemplifying Rinsp measurements taken on the various days indicated on the X axis and in the window W2.

Referring to the attached figure, a method for the early identification of exacerbations of COPD, in accordance with the present invention, comprises the steps of initiating 10 the procedure; measuring 11, with a predefined time frequency, a certain number of parameters that define pulmonary function and the respiratory pattern of a patient by means of the FOT technique; for each new measurement available, collecting 12 the parameters measured, thus constituting the corresponding time series thereof; verifying 13 whether the adaptation period, calculated from the beginning of the time series, has finished, i.e. evaluating whether the number of measurements collected is higher than a first predefined number - if not, start again from the beginning 10, and if so, eliminate 14 the abnormal values; verifying 15 whether the number of measurements in a given time period (having eliminated the abnormal values) is higher than a predefined number - if not, start again from the beginning 10, and if so, calculate 16 the time trend of said parameters in a predetermined time period; verifying 17 whether the trend of the latter, evaluated by using appropriate statistical methods or mathematical models, is significantly higher or lower than predefined numbers - if not, start again from the beginning 10, and if so, an impending exacerbation 18 has been predicted. Then start again from the beginning 10.

For the measurements 11 the patients are required to use an FOT device able to measure Rrs and Xrs separately during the inspiratory and expiratory phase, the derived parameters and the respiratory model parameters. Said device is composed of a generator of stimuli at low pressure (<5 cmH2O), a set of pressure and flow sensors, a patient interface, a respiration circuit and a calculation unit that operates the pressure generator, collects the data from the sensors and uses them to calculate the pulmonary impedance, the derived parameters and the respiratory pattern parameters according to specific algorithms. An embodiment example of said device is described by Gobbi et al (Gobbi, Milesi, Govoni, Pedotti & Dellacà 2009).

During each measurement, the patients are required to wear a nose plug and adopt systems to reduce vibration of the cheeks (for example, by supporting them using their hands) while they breathe spontaneously through the device, for example for two minutes or until a predefined number of breaths has been recorded.

The parameters that define the pulmonary function of a patient measured by means of the FOT technique are one or more of the following: inspiratory resistance (Rinsp) measured at a frequency ranging between 2 and 10Hz; inspiratory reactance (Xinsp) measured at a frequency ranging between 2 and 10Hz; difference between inspiratory and expiratory reactance ($\Delta$Xrs) measured at a frequency between 2 and 10Hz.

The respiratory pattern of a patient is described by the set of the following parameters: tidal volume ($V_T$), mean inspiratory (Ti) and expiratory times (Te), respiratory frequency (RR), respiratory duty cycle (Ti*RR), mean inspiratory (Vt/Ti) and expiratory flow (Vt/Te) and minute ventilation (Ve).

In one embodiment example of the method, the patient is required to perform one FOT measurement per day. The mean FOT and respiratory pattern parameters of each new daily measurement, calculated according to the intra-respiratory analysis method previously described, are collected 12 in the corresponding time series of the patient in question.

Since the measurement 11 requires the patient to breathe through the FOT device by means of a measurement interface, for example a mouthpiece, it is possible that the first measurements may not be usable due to adaptation of the patient to said interface. Said measurements should preferably be excluded. In one embodiment example of the method an adaptation period 13 of 8 days has been considered, so that the measurements contained in said time period are excluded from the following calculations. This passage is optional as it may not be necessary.

If an FOT measurement produces abnormal values, for example when carried out with an incorrect posture, without correct support of the cheeks, with a wrong positioning of the mouthpiece and/or of the nose plug, leaks around the measurement interface, due to obstruction of the filter by teeth or tongue, coughing, partial or total closure of the glottis, they must be eliminated 14 from the time series.

The method for detecting the abnormal values uses the normalized distance of one or more parameters calculated from the FOT measurement and the current daily respiratory pattern with corresponding median value, calculated from the measurements available within a time window of predefined length which includes the current and past FOT measurements.

In particular it was considered that if the value V of a given parameter, calculated as shown in the following equation, is higher than a threshold value TR, the current FOT measurement OP must be considered abnormal and therefore discarded.

$$ V = \frac{OP - m(OP(W1))}{m(OP(W1))} \geq TR \tag{1} $$

where:

m(OP(W1)) is considered the median of the values of a given parameter measured within the window W1, and W1 is a time window of predefined length containing the FOT measurements to be considered in the calculation, the new measurement and the past measurements.

Other approaches can be used to detect abnormal values in a time series of measurements and adapted for this application.

In a preferred embodiment of the present invention, the window W1 lasts 8 days and the threshold TR is equal to 0.5. The measurement is considered an abnormal value and will be ignored if the previous equation is verified for at least one of the following parameters: current volume $V_T$, inspiratory resistance Rinsp measured at 5 Hz, respiratory reactance Xinsp measured at 5Hz.

It is preferably checked 15 that, after removal of the abnormal values, at least a predefined number of measurements are present in a given time period W2, in order to have a significant number of measurements. In a preferred embodiment of the present invention, the time window W2 was chosen equal to 10 days and the minimum number of FOT measurements that must be present in W2 equal to 5.

It is checked that in W2 there are at least X% measurements. For example, if X% = 50% and W2 = 10 days, it must be checked that there are at least 5 measurements in W2.

The trends of all or a part of the FOT parameters and respiratory model are then calculated 16, by means of appropriate statistical methods or mathematical models and starting from the measurements available in the same time period W2. For example, a trend could be quantified, for each parameter in question, by means of an N order polynomial regression model relative to the measurements performed and previously processed considering: 1) the coefficients of the polynomial equation calculated ($ß_0$ for the known term, $ß_1$ for the coefficient of the first degree term, and so on), 2) the statistical significances (p-value) of each coefficient against the null hypothesis of being equal to zero, and 3) the correlation coefficient of the polynomial regression ($r^2$).

For example, a linear regression model and the parameters Rinsp, Xinsp and DeltaXrs can be used, thus calculating $ß1_{Rinsp}$, $ß1_{Xinsp}$ and $ß1_{deltaXrs}$.

For each FOT parameter considered, it is evaluated whether the statistical regression model identifies a progression, calculating the probability of one or more parameters of the model ß1 being different from zero, comparing said probability (also known as p-value), with a threshold, for example p<0.05. If this criterion is verified, it can be affirmed that the statistical model describes the progression of the parameter FOT sustained over time.

The overall goodness of the regression is then evaluated and its physiological significance. For measurement of the goodness of the regression, the correlation coefficient r2 can, for example, be used, which must be greater than a given threshold. The physiological significance of the regression is evaluated through a criterion applied to ß1, which depends in turn on the FOT parameter considered. In this example, the criteria associated with the respective coefficients ß1 are: $ß1_{Rinsp} >0$, $ß1_{Xinsp} <0$, $ß1_{deltaXrs} >0$.

If the statistical regression model identifies a progression for a given FOT parameter and, simultaneously, the regression has a valid physiological significance and a high goodness level, the method assigns a value 1 to a corresponding trend parameter MI, which otherwise remains = 0.

Therefore, for every parameter analysed, the trend is considered in the direction of worsening of the pathology if it is above or below a predefined threshold. If so, a value 1 is assigned to a corresponding trend parameter, $MI_P$. If not, the corresponding trend parameter $MI_P$ is maintained at 0.

For example, we will therefore have three trend parameters $MI_{Rinsp}$, $MI_{Xinsp}$ and $MI_{deltaXrs}$ and each of them can assume the value 1 or remain at 0.

Lastly, a exacerbation is scheduled by applying the following equation (2) which calculates a weighted sum of the trend parameters just processed:

$$\sum_p MI_p * w_p \geq TH \qquad\qquad (2)$$

where $W_P$ ($0 \leq W_P \leq 1$) is a weight associated with the trend parameter $MI_P$ of the parameter p in question and TH is a threshold.

In a preferred embodiment of the invention a linear regression model was applied (with N = 1) to each of the following parameters: inspiratory resistance (Rinsp) measured at 5 Hz, absolute value of the inspiratory reactance (Xinsp) measured at 5 Hz, difference between inspiratory and expiratory reactance (ΔXrs) measured at 5 Hz.

Furthermore, for every parameter a value equal to 1 is assigned to the corresponding trend parameter $MI_P$ if all the following conditions have been verified for the following values: the absolute value of the coefficient $ß_1$ (slope of the regression line) must be greater than 0, the corresponding p-value must be less than 0.05 and the correlation coefficient of the polynomial regression ($r^2$) must be greater than 0.4.

In one embodiment example of the present invention, the measurements performed on the patient are transferred to a microprocessor which carries out all the processing operations, according to the predefined program, and provides the final results to a viewer, identifying, in automatic mode, the presence of exacerbations of chronic obstructive pulmonary disease.

An impending exacerbation was identified using the weights $W_P$ equal to 1 and the predefined threshold TH equal to 1, i.e. if the value calculated was greater than or equal to 1 as in the following equation:

$$1 * MI_{Rinsp} + 1 * MI_{Xinsp} + 1 * MI_{\Delta xrs} \geq 1$$

The Applicant performed a test on 24 patients for 8 months taking daily measurements by means of FOT using a commercial instrument.

The characteristics of the 24 COPD patients monitored are shown in Table 1.

Throughout the study the patients were telephonically interviewed once a week to collect the following information: prescriptions and use of drugs and/or antibiotics, non-scheduled medical examinations and admissions to hospital.

The exacerbations were classified as:

Slight: where there were changes in the current treatment or prescription of a short-acting bronchodilator,
Intermediate: where a corticosteroid was prescribed,
Severe: where systemic antibiotics were prescribed,
Very serious: when the patient was admitted to hospital.

In order to evaluate the performances of this method, all exacerbations were grouped together, regardless of their severity. Furthermore, a sub-analysis was carried out only on severe and very serious exacerbations, since the latter are considered the most critical events in terms of both the patient and the health service.

During the monitoring period, the patients reported a total of 26 exacerbations, 13 of which were of slight or intermediate type, and 13 of severe or serious type. Of these, 18 (69%) were correctly identified by the method described above. Eight exacerbations of slight or intermediate type (61.5%) and 10 exacerbations of severe or very serious type (77%) were correctly identified by the method described above.

| TABLE 1 | |
|---|---|
| Sex (M/F) | 20/4 |

(continued)

| TABLE 1 | |
|---|---|
| Age (years) | $72.3 \pm 6.9$ |
| Height (cm) | $156.8 \pm 7.0$ |
| Weight (kg) | $74.9 \pm 14.5$ |
| Body mass index BMI (kg/m2) | $26.5 \pm 4.3$ |
| Maximum expiratory volume in 1 according to FEV1 (I) | $1.1 \pm 0.3$ |
| FEV1 (%pred) | $41.3 \pm 12.4$ |
| FEV1/FVC (%pred) | $42.1 \pm 11.9$ |

**Claims**

1. A system for the early identification of exacerbation of chronic obstructive pulmonary disease, comprising a microprocessor adapted to perform the following steps:

   - measuring, with a plurality of measurements at predefined time frequency, a first set of parameters that define the pulmonary function of a patient by means of the forced oscillation technique (FOT), represented by one or more of the following: inspiratory resistance (Rinsp) measured at a frequency ranging between 2 and 10Hz; inspiratory reactance (Xinsp) measured at a frequency ranging between 2 and 10Hz; difference between inspiratory and expiratory reactance ($\Delta$Xrs) measured at a frequency ranging between 2 and 10Hz;
   - measuring simultaneously a second set of parameters indicative of the respiratory pattern, represented by at least one or more of the following: tidal volume (VT), mean inspiratory (Ti) and expiratory times (Te), respiratory frequency (RR), respiratory duty cycle (Ti*RR), mean inspiratory (Vt/Ti) and expiratory flow (Vt/Te) and a minute ventilation (Ve);
   - eliminating the measurements affected by artifacts containing abnormal values in at least one parameter of each of the first and the second sets of parameters, by the step of comparing each measurement with a corresponding median value, calculated from the measurements available within a predetermined time window, including the current and past measurements;
   - calculating the trends of the first set of parameters and the second set of parameters in a predefined time period by calculation of an N order polynomial regression model;
   identifying an impending exacerbation by comparing parameters describing said trends of the first set of parameters and the second set of parameters by comparing at least one coefficient ($\beta$) of the N order polynomial regressions with predefined thresholds;
   wherein for said first and second sets of parameters the deterioration trend of the pathology is assessed according to whether 1) at least one of its polynomial regression coefficients ($\beta$), 2) its corresponding p-value against the null hypothesis of being equal to zero and 3) the correlation coefficient of the polynomial regression ($r^2$) are above or below a predefined threshold and depending on this assessment, assigning a value 1 or 0 to a corresponding trend parameter ($MI_p$); and
   predicting an exacerbation by performing a weighted sum of said trend parameters ($MI_P$).

2. The system according to claim 1 **characterized in that** the measurement of the pulmonary function of a patient by means of the forced oscillation technique (FOT) is performed at least once every two days.

3. The system according to claim 1 **characterized in that** the step of eliminating the measurements containing abnormal values comprises the step of eliminating all the measurements taken in a given time period from the beginning of the measurements.

4. The system according to claim 1 **characterized in that** after the step of eliminating the measurements containing abnormal values, there is the step of verifying whether the number of remaining measurements is higher than a predefined number.

5. The system according to claim 1 **characterized in that** the step of eliminating the abnormal values of the above-mentioned parameters comprises the step of considering that if the value V of a given parameter, calculated as shown in the following equation, is higher than a threshold value TR, the current FOT measurement OP must be considered abnormal and therefore discarded as the following equation

$$V = \frac{OP - m(OP(W1))}{m(OP(W1))} \geq TR$$

where:

m (OP(W1)) is considered the median of the values of a given parameter measured within the window W1, and W1 is a time window of predefined length containing the FOT measurements to be considered in the calculation.

6. The system according to claim 1 **characterized in that** the step of predicting a exacerbation of chronic obstructive pulmonary disease by performing a weighted sum of said trend parameters is calculated as

$$\sum_{p} MIp * Wp \geq TH$$

where $W_P$ ($0<W_P<1$) is a weight associated with the trend parameter $MI_P$ of the parameter p in question and TH is a threshold.

7. A computer program adapted to perform a method for the early identification of exacerbation of chronic obstructive pulmonary disease when run on a microprocessor of the system according to claim 1, the method comprising the steps of:

- measuring, with a plurality of measurements at predefined time frequency, a first set of parameters that define the pulmonary function of a patient by means of the forced oscillation technique (FOT), represented by one or more of the following: inspiratory resistance (Rinsp) measured at a frequency ranging between 2 and 10Hz; inspiratory reactance (Xinsp) measured at a frequency ranging between 2 and 10Hz; difference between inspiratory and expiratory reactance (ΔXrs) measured at a frequency ranging between 2 and 10Hz;
- measuring simultaneously a second set of parameters indicative of the respiratory pattern, represented by at least one or more of the following: tidal volume (VT), mean inspiratory (Ti) and expiratory times (Te), respiratory frequency (RR), respiratory duty cycle (Ti*RR), mean inspiratory (Vt/Ti) and expiratory flow (Vt/Te) and a minute ventilation (Ve);
- eliminating the measurements affected by artifacts containing abnormal values in at least one parameter of each of the first and the second sets of parameters, by the step of comparing each measurement with a corresponding median value, calculated from the measurements available within a predetermined time window, including the current and past measurements;
- calculating the trends of the first set of parameters and the second set of parameters in a predefined time period by calculation of an N order polynomial regression model;
identifying an impending exacerbation by comparing parameters describing said trends of the first set of parameters and the second set of parameters by comparing at least one coefficient (β) of the N order polynomial regressions with predefined thresholds;
wherein for said first and second sets of parameters the deterioration trend of the pathology is assessed according to whether 1) at least one of its polynomial regression coefficients (β), 2) its corresponding p-value against the null hypothesis of being equal to zero and 3) the correlation coefficient of the polynomial regression (r2) are above or below a predefined threshold and depending on this assessment, assigning a value 1 or 0 to a corresponding trend parameter (Mlp); and
predicting an exacerbation by performing a weighted sum of said trend parameters (MIP).

**Patentansprüche**

1. Ein System zur frühen Identifizierung einer Verschlimmerung einer chronischen obstruktiven Lungenerkrankung, beinhaltend einen Mikroprozessor, der angepasst ist, um die folgenden Schritte durchzuführen:

- Messen, mit einer Vielzahl von Messungen bei einer vordefinierten Zeitfrequenz, eines ersten Satzes von Parametern, die die Lungenfunktion eines Patienten mittels der forcierten Oszillationstechnik (FOT) definieren, dargestellt durch eines oder mehrere der Folgenden: inspiratorischer Widerstand (Rinsp), gemessen bei einer Frequenz, die zwischen 2 und 10 Hz liegt; inspiratorische Reaktanz (Xinsp), gemessen bei einer Frequenz, die zwischen 2 und 10 Hz liegt; Differenz zwischen inspiratorischer und exspiratorischer Reaktanz (ΔXrs), gemessen bei einer Frequenz, die zwischen 2 und 10 Hz liegt;

- gleichzeitiges Messen eines zweiten Satzes von Parametern, die das Atemmuster anzeigen, dargestellt durch mindestens eines oder mehrere der Folgenden: Atemzugvolumen (VT), mittlere inspiratorische (Ti) und exspiratorische Zeiten (Te), Atemfrequenz (RR), Atemarbeitszyklus (Ti*RR), mittlere inspiratorische (Vt/Ti) und exspiratorische Strömung (Vt/Te) und eine Minutenventilation (Ve);

- Eliminieren der Messungen, die von Artefakten betroffen sind, die anormale Werte in mindestens einem Parameter von jedem des ersten und des zweiten Satzes von Parametern enthalten, durch den Schritt des Vergleichens jeder Messung mit einem entsprechenden Medianwert, berechnet aus den Messungen, die innerhalb eines vorbestimmten Zeitfensters verfügbar sind, einschließlich der aktuellen und vergangenen Messungen;

- Berechnen der Trends des ersten Satzes von Parametern und des zweiten Satzes von Parametern in einer vordefinierten Zeitperiode durch Berechnung eines Polynomregressionsmodells N-ter Ordnung;

Identifizieren einer bevorstehenden Verschlimmerung durch Vergleichen von Parametern, die die Trends des ersten Satzes von Parametern und des zweiten Satzes von Parametern beschreiben, durch Vergleichen mindestens eines Koeffizienten (β) der Polynomregressionen N-ter Ordnung mit vordefinierten Schwellenwerten;

wobei für den ersten und den zweiten Satz von Parametern der Verschlechterungstrend der Pathologie in Abhängigkeit davon beurteilt wird, ob 1) mindestens einer seiner Polynomregressionskoeffizienten (β), 2) sein entsprechender p-Wert gegen die Nullhypothese, gleich null zu sein, und 3) der Korrelationskoeffizient der Polynomregression ($r^2$) über oder unter einem vordefinierten Schwellenwert liegen, und in Abhängigkeit von dieser Beurteilung Zuweisen eines Werts 1 oder 0 zu einem entsprechenden Trendparameter ($MI_p$); und Vorhersagen einer Verschlimmerung durch Durchführen einer gewichteten Summe der Trendparameter ($MI_p$).

2. System gemäß Anspruch 1, **dadurch gekennzeichnet, dass** die Messung der Lungenfunktion eines Patienten mittels der forcierten Oszillationstechnik (FOT) mindestens einmal alle zwei Tage durchgeführt wird.

3. System gemäß Anspruch 1, **dadurch gekennzeichnet, dass** der Schritt des Eliminierens der Messungen, die anormale Werte enthalten, den Schritt des Eliminierens aller Messungen umfasst, die in einer gegebenen Zeitperiode ab dem Beginn der Messungen genommen wurden.

4. System gemäß Anspruch 1, **dadurch gekennzeichnet, dass** es nach dem Schritt des Eliminierens der Messungen, die anormale Werte enthalten, den Schritt des Überprüfens gibt, ob die Anzahl der verbleibenden Messungen höher als eine vordefinierte Anzahl ist.

5. System gemäß Anspruch 1, **dadurch gekennzeichnet, dass** der Schritt des Eliminierens der anormalen Werte der oben genannten Parameter den Schritt des Berücksichtigens umfasst, dass, wenn der Wert V eines gegebenen Parameters, berechnet wie in der folgenden Gleichung gezeigt, höher als ein Schwellenwert TR ist, die aktuelle FOT-Messung OP als anormal betrachtet werden muss und daher als die folgende Gleichung verworfen werden muss

$$V = \frac{OP - m(OP(W1))}{m(OP(W1))} \geq TR$$

wobei:

m(OP(W1)) als der Median der Werte eines gegebenen Parameters berücksichtigt wird, die innerhalb des Fensters W1 gemessen werden, und
W1 ein Zeitfenster von vordefinierter Länge ist, das die FOT-Messungen enthält, die bei der Berechnung zu berücksichtigen sind.

6. System gemäß Anspruch 1, **dadurch gekennzeichnet, dass** der Schritt des Vorhersagens einer Verschlimmerung einer chronischen obstruktiven Lungenerkrankung durch Durchführen einer gewichteten Summe der Trendparameter berechnet wird als

$$\sum_{p} Mlp * Wp \geq TH$$

wobei $W_P(O<W_P<1)$ ein Gewicht ist, das mit dem Trendparameter $MI_P$ des betreffenden Parameters p assoziiert ist, und TH ein Schwellenwert ist.

**7.** Ein Computerprogramm, das angepasst ist, um ein Verfahren zur frühen Identifizierung einer Verschlimmerung einer chronischen obstruktiven Lungenerkrankung durchzuführen, wenn es auf einem Mikroprozessor des Systems gemäß Anspruch 1 ausgeführt wird, wobei das Verfahren die folgenden Schritte beinhaltet:

- Messen, mit einer Vielzahl von Messungen bei einer vordefinierten Zeitfrequenz, eines ersten Satzes von Parametern, die die Lungenfunktion eines Patienten mittels der forcierten Oszillationstechnik (FOT) definieren, dargestellt durch eines oder mehrere der Folgenden: inspiratorischer Widerstand (Rinsp), gemessen bei einer Frequenz, die zwischen 2 und 10 Hz liegt; inspiratorische Reaktanz (Xinsp), gemessen bei einer Frequenz, die zwischen 2 und 10 Hz liegt; Differenz zwischen inspiratorischer und exspiratorischer Reaktanz (ΔXrs), gemessen bei einer Frequenz, die zwischen 2 und 10 Hz liegt;
- gleichzeitiges Messen eines zweiten Satzes von Parametern, die das Atemmuster anzeigen, dargestellt durch mindestens eines oder mehrere der Folgenden: Atemzugvolumen (VT), mittlere inspiratorische (Ti) und exspiratorische Zeiten (Te), Atemfrequenz (RR), Atemarbeitszyklus (Ti*RR), mittlere inspiratorische (Vt/Ti) und exspiratorische Strömung (Vt/Te) und eine Minutenventilation (Ve);
- Eliminieren der Messungen, die von Artefakten betroffen sind, die anormale Werte in mindestens einem Parameter von jedem des ersten und des zweiten Satzes von Parametern enthalten, durch den Schritt des Vergleichens jeder Messung mit einem entsprechenden Medianwert, berechnet aus den Messungen, die innerhalb eines vorbestimmten Zeitfensters verfügbar sind, einschließlich der aktuellen und vergangenen Messungen;
- Berechnen der Trends des ersten Satzes von Parametern und des zweiten Satzes von Parametern in einer vordefinierten Zeitperiode durch Berechnung eines Polynomregressionsmodells N-ter Ordnung;
Identifizieren einer bevorstehenden Verschlimmerung durch Vergleichen von Parametern, die die Trends des ersten Satzes von Parametern und des zweiten Satzes von Parametern beschreiben, durch Vergleichen mindestens eines Koeffizienten (β) der Polynomregressionen N-ter Ordnung mit vordefinierten Schwellenwerten;
wobei für den ersten und den zweiten Satz von Parametern der Verschlechterungstrend der Pathologie in Abhängigkeit davon beurteilt wird, ob 1) mindestens einer seiner Polynomregressionskoeffizienten (β), 2) sein entsprechender p-Wert gegen die Nullhypothese, gleich null zu sein, und 3) der Korrelationskoeffizient der Polynomregression (r2) über oder unter einem vordefinierten Schwellenwert liegen, und in Abhängigkeit von dieser Beurteilung Zuweisen eines Werts 1 oder 0 zu einem entsprechenden Trendparameter (MIp); und
Vorhersagen einer Verschlimmerung durch Durchführen einer gewichteten Summe der Trendparameter (MiP).

**Revendications**

**1.** Un système pour l'identification précoce de l'exacerbation d'une maladie pulmonaire obstructive chronique, comprenant un microprocesseur conçu pour effectuer les étapes suivantes :

- la mesure, avec une pluralité de mesures à une fréquence temporelle prédéfinie, d'un premier ensemble de paramètres qui définissent la fonction pulmonaire d'un patient au moyen de la technique d'oscillation forcée (FOT), représentée par un ou plusieurs des éléments suivants : la résistance inspiratoire (Rinsp) mesurée à une fréquence comprise entre 2 et 10 Hz ; la réactance inspiratoire (Xinsp) mesurée à une fréquence comprise entre 2 et 10 Hz ; la différence entre la réactance inspiratoire et expiratoire (ΔXrs) mesurée à une fréquence comprise entre 2 et 10 Hz ;
- la mesure simultanée d'un deuxième ensemble de paramètres indicatifs du schéma respiratoire, représenté par au moins un ou plusieurs des éléments suivants : le volume respiratoire (VT), les temps inspiratoire (Ti) et expiratoire (Te) moyens, la fréquence respiratoire (RR), le cycle de service respiratoire (Ti*RR), le flux inspiratoire (Vt/Ti) et expiratoire (Vt/Te) moyen et une ventilation minute (Ve) ;
- l'élimination des mesures affectées par des artefacts contenant des valeurs anormales dans au moins un paramètre de chacun des premier et deuxième ensembles de paramètres, par l'étape de comparaison de chaque mesure avec une valeur médiane correspondante, calculée à partir des mesures disponibles dans une fenêtre temporelle prédéterminée, incluant les mesures actuelles et passées ;

- le calcul des tendances du premier ensemble de paramètres et du deuxième ensemble de paramètres dans une période temporelle prédéfinie par le calcul d'un modèle de régression polynomiale d'ordre N ;

l'identification d'une exacerbation imminente par la comparaison de paramètres décrivant lesdites tendances du premier ensemble de paramètres et du deuxième ensemble de paramètres par la comparaison d'au moins un coefficient ($\beta$) des régressions polynomiales d'ordre N avec des seuils prédéfinis ;

dans lequel pour lesdits premier et deuxième ensembles de paramètres, la tendance de détérioration de la pathologie est évaluée selon que 1) au moins l'un de ses coefficients de régression polynomiale ($\beta$), 2) sa valeur p correspondante par rapport à l'hypothèse nulle d'être égal à zéro et 3) le coefficient de corrélation de la régression polynomiale ($r^2$) sont supérieurs ou inférieurs à un seuil prédéfini et en fonction de cette évaluation, l'attribution d'une valeur 1 ou 0 à un paramètre de tendance correspondant ($MI_p$) ; et

la prédiction d'une exacerbation en effectuant une somme pondérée desdits paramètres de tendance ($MI_p$).

2. Le système selon la revendication 1 **caractérisé en ce que** la mesure de la fonction pulmonaire d'un patient au moyen de la technique d'oscillation forcée (FOT) est effectuée au moins une fois tous les deux jours.

3. Le système selon la revendication 1 **caractérisé en ce que** l'étape d'élimination des mesures contenant des valeurs anormales comprend l'étape d'élimination de toutes les mesures prises dans une période temporelle donnée à partir du début des mesures.

4. Le système selon la revendication 1 **caractérisé en ce qu'**après l'étape d'élimination des mesures contenant des valeurs anormales, il y a l'étape de vérification si le nombre de mesures restantes est plus élevé qu'un nombre prédéfini.

5. Le système selon la revendication 1 **caractérisé en ce que** l'étape d'élimination des valeurs anormales des paramètres susmentionnés comprend l'étape de considération que si la valeur V d'un paramètre donné, calculée comme montré dans l'équation suivante, est plus élevée qu'une valeur seuil TR, la mesure FOT actuelle OP doit être considérée comme anormale et donc rejetée comme l'équation suivante

$$V = \frac{OP - m(OP(W1))}{m(OP(W1))} \geq TR$$

où :

m (OP(W1)) est considéré comme la médiane des valeurs d'un paramètre donné mesurées dans la fenêtre W1, et W1 est une fenêtre temporelle de longueur prédéfinie contenant les mesures FOT à considérer dans le calcul.

6. Le système selon la revendication 1 **caractérisé en ce que** l'étape de prédiction d'une exacerbation d'une maladie pulmonaire obstructive chronique en effectuant une somme pondérée desdits paramètres de tendance est calculée comme

$$\sum_p MIp * Wp \geq TH$$

où $W_P$ (0<Wp<1) est un poids associé au paramètre de tendance $MI_p$ du paramètre p en question et TH est un seuil.

7. Un programme informatique conçu pour effectuer un procédé pour l'identification précoce de l'exacerbation d'une maladie pulmonaire obstructive chronique lorsqu'il est exécuté sur un microprocesseur du système selon la revendication 1, le procédé comprenant les étapes consistant en :

- la mesure, avec une pluralité de mesures à une fréquence temporelle prédéfinie, d'un premier ensemble de paramètres qui définissent la fonction pulmonaire d'un patient au moyen de la technique d'oscillation forcée (FOT), représentée par un ou plusieurs des éléments suivants : la résistance inspiratoire (Rinsp) mesurée à une fréquence comprise entre 2 et 10 Hz ; la réactance inspiratoire (Xinsp) mesuré à une fréquence comprise entre 2 et 10 Hz ; la différence entre la réactance inspiratoire et expiratoire ($\Delta$Xrs) mesurée à une fréquence comprise entre 2 et 10 Hz ;

- la mesure simultanée d'un deuxième ensemble de paramètres indicatifs du schéma respiratoire, représenté par au moins un ou plusieurs des éléments suivants : le volume respiratoire (VT), les temps inspiratoire (Ti) et expiratoire (Te) moyens, la fréquence respiratoire (RR), le cycle de service respiratoire (Ti*RR), le flux inspiratoire (Vt/Ti) et expiratoire (Vt/Te) moyen et une ventilation minute (Ve) ;

- l'élimination des mesures affectées par des artefacts contenant des valeurs anormales dans au moins un paramètre de chacun des premier et deuxième ensembles de paramètres, par l'étape de comparaison de chaque mesure avec une valeur médiane correspondante, calculée à partir des mesures disponibles dans une fenêtre temporelle prédéterminée, incluant les mesures actuelles et passées ;

- le calcul des tendances du premier ensemble de paramètres et du deuxième ensemble de paramètres dans une période temporelle prédéfinie par le calcul d'un modèle de régression polynomiale d'ordre N ;

l'identification d'une exacerbation imminente par la comparaison de paramètres décrivant lesdites tendances du premier ensemble de paramètres et du deuxième ensemble de paramètres par la comparaison d'au moins un coefficient (β) des régressions polynomiales d'ordre N avec des seuils prédéfinis ;

dans lequel pour lesdits premier et deuxième ensembles de paramètres, la tendance de détérioration de la pathologie est évaluée selon que 1) au moins l'un de ses coefficients de régression polynomiale (β), 2) sa valeur p correspondante par rapport à l'hypothèse nulle d'être égal à zéro et 3) le coefficient de corrélation de la régression polynomiale (r2) sont supérieurs ou inférieurs à un seuil prédéfini et en fonction de cette évaluation, l'attribution d'une valeur 1 ou 0 à un paramètre de tendance correspondant (MIp) ; et

la prédiction d'une exacerbation en effectuant une somme pondérée desdits paramètres de tendance (MiP).

Fig. 1

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2245985 A1
- EP 2613696 A1

**Non-patent literature cited in the description**

- **GULOTTA et al.** *AJRCCM*, 2012
- **DELLACÀ et al.** *ERJ*, 2004
- **GOBBI et al.** *IEEE Telemed*, 2009
- **GOBBI** ; **MILESI** ; **GOVONI**. *Pedotti & Dellacà*, 2009